(19) Europäisches Patentamt
European Patent Office
Office européen des brevets

(11) Numéro de publication: **0 003 383**
**B1**

(12) **FASCICULE DE BREVET EUROPEEN**

(45) Date de publication du fascicule du brevet: **09.02.83**

(21) Numéro de dépôt: **79200037.4**

(22) Date de dépôt: **22.01.79**

(51) Int. Cl.$^3$: **C 07 D 213/74,**
C 07 D 401/12,
C 07 D 413/12,
A 61 K 31/44

(54) **Dérivés de 4-amino-3-sulfonamido-pyridine.**

(30) Priorité: **31.01.78 GB 391878**

(43) Date de publication de la demande:
**08.08.79 Bulletin 79/16**

(45) Mention de la délivrance du brevet:
**09.02.83 Bulletin 83/6**

(84) Etats contractants désignés:
**DE NL SE**

(56) Documents cités:
**FR - A - 2 073 343**
**FR - A - 2 113 927**
**FR - A - 2 194 426**
**FR - A - 2 267 775**

**Le dossier contient des informations techniques présentées postérieurement au dépôt de la demande et ne figurant pas dans le présent fascicule.**

(73) Titulaire: **A. CHRISTIAENS SOCIETE ANONYME**
**60, rue de l'Etuve**
**B-1000 Bruxelles (BE)**

(72) Inventeur: **Lapiere, Charles**
**Driekruisenstraat 75**
**B-3700 Tongeren (BE)**
Inventeur: **Delarge, Jacques**
**Haie des Chênes 7**
**B-4052 Dolembreux (BE)**
Inventeur: **Thunus, Léopold**
**rue Saint Léonard 227**
**B-4000 Liège (BE)**
Inventeur: **Georges, André**
**rue du petit Ry 97**
**B-1340 Ottignies (BE)**
Inventeur: **De Ridder, René**
**Avenue Lambeau 31**
**B-1200 Bruxelles (BE)**
Inventeur: **Ghys, Arlette**
**Avenue Maréchal Joffre 107**
**B-1190 Bruxelles (BE)**

(74) Mandataire: **De Brabanter, Maurice et al,**
**Bureau VANDER HAEGHEN 63 Avenue de la Toison d'Or**
**B-1060 Bruxelles (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 003 383 B1

Dérivés de 4-amino-3-sulfonamido-pyridine

La présente invention concerne de nouveaux dérivés de la pyridine, leur préparation et leur utilisation.

Ces nouveaux dérivés de la pyridine sont des composés de la formule générale

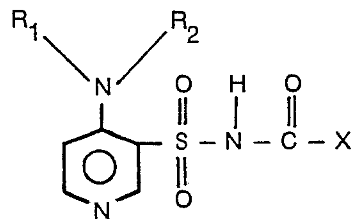

(I)

dans laquelle

$R_1$ désigne un atome d'hydrogène, un radical alkyle en $C_1$—$C_4$ à chaîne droite ou ramifiée ou le radical cyclohexyle, et

$R_2$ représente un radical alkyle en $C_1$—$C_7$ à chaîne droite ou ramifiée, un groupe chloroalkyle en $C_1$—$C_3$, un radical cycloalkyle en $C_3$—$C_8$, le radical propényle ou propynyle, un radical phényle, éventuellement substitué par un groupe méthyle, par un atome de chlore ou par un groupe trifluorométhyle, le radical benzyle, le radical 1-méthyl 2-phényl éthyle ou le radical norbornyle;

et X représente un radical de formule:

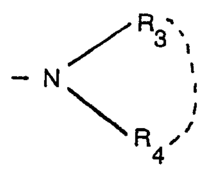

(IV)

où $R_3$ représente un atome d'hydrogène, un radical alkyle en $C_2$—$C_4$ à chaîne droite ou ramifiée ou un groupe propényle et

$R_4$ un radical alkyle en $C_1$—$C_4$ à chaîne droite ou ramifiée, un groupe propényle, hydroxyéthyle ou benzyle, ou $R_3$ et $R_4$ forment avec l'atome d'azote adjacent un groupe hétérocyclique azoté saturé en $C_4$—$C_6$ pouvant contenir un deuxième atome d'azote ou un autre hétéroatome et éventuellement substitué par un ou deux groupes méthyle ou un groupe phényle ou chlorophényle, étant entendu que:

—$R_1$ et $R_2$ peuvent prendre toutes les valeurs indiquées lorsque le radical

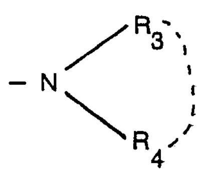

représente une fonction amine tertiaire, hétérocyclique ou di-N-substituée par des groupes en $C_2$ au moins, et

—$R_1$, $R_3$ et $R_4$ peuvent prendre toutes les valeurs indiquées lorsque $R_2$ représente un radical alkyle en $C_5$—$C_7$ à chaîne droite ou ramifiée, un radical cycloalkyle en $C_3$—$C_8$, un groupe chloroalkyle en $C_1$—$C_3$ ou le groupe norbornyle; ainsi que les sels d'addition d'acides physiologiquement acceptables de ces composés.

La littérature antérieure cite des dérivés de 4-amino-3-sulfonamido-pyridine pour lesquels une activité diurétique est rapportée.

Ainsi, le brevet FR—A—2 073 343 décrit des 4-(N-méthyl et/ou N-phényl)-amino-3-sulfamoyl-pyridines; le brevet FR—A—2 194 426 décrit des 4-phénylamino-3-acylsulfonamido pyridines et le brevet FR—A—2 267 775 décrit des composés 4-(N-alkyl-$C_1$—$C_4$ et/ou N-phényl)-amino-3-carbamoyl-sulfonamido-pyridines à fonction carbamoyle secondaire.

Les composés de formule I suivant l'invention peuvent être préparés comme suit:

Pour la préparation d'un composé de la formule générale I, pour lequel $R_1$ et $R_2$ possèdent les significations définies et X représente un groupe

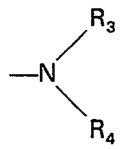

où $R_3$=H et $R_4$ possède l'une des significations définies, le procédé consiste à faire réagir un composé de la formule

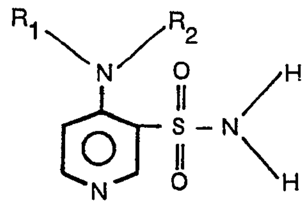

(V)

dans laquelle $R_1$ et $R_2$ possèdent les significations définies, avec un isocyanate de la formule

$$R_4N=C=O \qquad (VI)$$

où $R_4$ possède l'une des significations définies.

Cette réaction peut être réalisée en présence de triéthylamine ou en utilisant le sel de sodium du pyridine-sulfonamide de la formule V.

Pour la préparation d'un composé de la formule générale I, dans laquelle $R_1$=H et $R_2$ désigne un groupe hétérocyclique azoté saturé, tandis que X représente un groupe de la formule

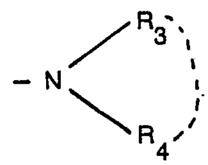

où $R_3$=H et $R_4$ possède l'une des significations définies, on fait réagir l'acide 3-sulfonamide-pyridine 4-sulfonique de la formule

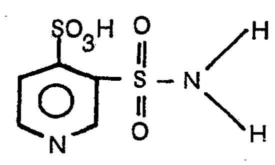

(IX)

d'abord avec une N-amino-amine hétérocyclique saturée telle que la 1-amino-pipéridine, puis le composé intermédiaire formé, de la formule V avec $R_1$=H et $R_2$=groupe hétérocyclique azoté saturé, avec un isocyanate de la formule VI, dans laquelle $R_4$ possède l'une des significations définies.

Pour la préparation d'un composé de la formule générale I, pour lequel $R_1$ et $R_2$ possèdent les significations définies et X représente un groupe

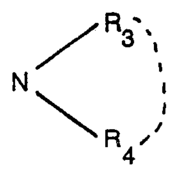,

où $R_3$ et $R_4$ possèdent les significations définies, on fait réagir un composé de la formule

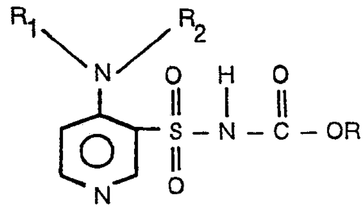

(X)

dans laquelle $R_1$ et $R_2$ possèdent les significations définies et R désigne un radical alkyle, avec une amine de la formule

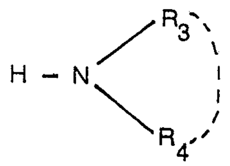

(XI)

Cette réaction est de préférence réalisée en faisant réagir le pyridine-sulfonylcarbamate de la formule X avec un excès de l'amine de la formule XI dans un solvant organique, en particulier le toluène ou le chloroforme, et en présence d'un tamis moléculaire de 4 A, soit à la température de reflux du solvant ou à une température plus élevée dans un autoclave.

Pour la préparation d'un composé de la formule générale I, dans laquelle $R_1$=H, $R_2$ possède les significations, définies et X désigne un groupe de la formule

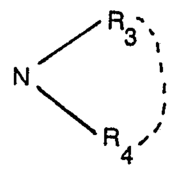

dans laquelle $R_3$ et $R_4$ possèdent les significations définies, le procédé consiste à faire réagir un 1,1-dioxyde de 3-oxo-3,4-dihydro-1,2,4-pyrido[4,3-e]-thiadiazine, de la formule

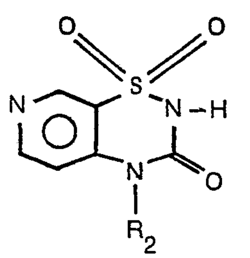

(XII)

dans laquelle $R_2$ possède l'une des significations définies, avec un excès d'une amine de la formule XI, dans laquelle $R_3$ et $R_4$ possèdent les significations définies.

Cette réaction est de préférence effectuée dans un solvant organique, en particulier le toluène, et à la température de reflux du milieu réactionnel.

Pour la préparation d'un composé de la formule générale X, dans laquelle $R_1$ et $R_2$ possèdent les significations définies et R représente un radical alkyle, le procédé consiste à faire réagir un composé de la formule générale V, dans laquelle $R_1$ et $R_2$ possèdent les significations définies, avec un chloroformiate d'alkyle de la formule

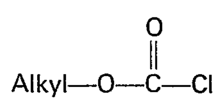

(XIII).

Les 4-amino-3-sulfonamido-pyridines de la formule V, employées comme composés de départ dans certains de ces procédés peuvent être préparées à partir de la 3-sulfonamido-4-chloropyridine de la formule

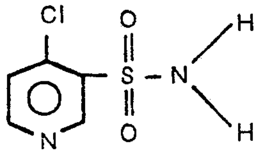

(XIV)

par l'un des modes opératoires ci-après.

*Mode opératoire A*

On fait réagir la 3-sulfonamido-4-chloropyridine avec un excès d'une amine de la formule

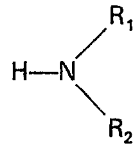

(XV)

dans laquelle $R_1$ et $R_2$ possèdent les significations définies, dans un solvant approprié, tel que l'éthanol, l'isopropanol et le propylène-glycol, à la température de reflux du mélange réactionnel, ou dans un autoclave à 140°C en employant l'éthanol comme solvant.

*Mode opératoire B*

On fait réagir la 3-sulfonamido-4-chloropyridine et un excès d'une amine de la formule XV, dans laquelle $R_1$ et $R_2$ possèdent les significations définies, en chauffant le mélange des deux réactifs à son point de fusion.

Les composés de la formule générale I se sont avérés posséder des propriétés diurétiques intéressantes. Ces propriétés ont été étudiées à l'aide de la procédure décrite ci-après.

Des lots de trois rats, d'un poids de 150 à 200 g, sont constitués au hasard, les animaux de chaque lot recevant un traitement identique. Le composé à examiner est administré par gavage gastrique à une dose de 50 mg/kg sous forme d'une solution ou d'une suspension aqueuse, contenant 0,45% de méthylcellulose (une substance mucilagineuse inerte); aux animaux témoins, on administre le véhicule aqueux en tant que placébo.

A tous les animaux, on administre simultanément 25 ml/kg de solution physiologique par injection souscutanée.

Les animaux constituant un lot sont enfermés dans la même cage et leurs urines sont récoltées pendant 4 heures. L'élimination urinaire ou diurèse est exprimée en ml/kg de poids des animaux et la comparaison du volume éliminé par les animaux traités avec celui des animaux témoins permet d'estimer l'action diurétique des composés examinés. Les résultats obtenus sont résumés dans le tableau ci-après.

TABLEAU I

Résultats pharmacologiques

| Composés de l'exemple | Diurèse ml/kg/4h |
|---|---|
| 3 | 95 |
| 4 | 87 |
| 5 | 96 |
| 6 | 109 |
| 29 | 88 |
| 30 | 87 |
| 37 | 111 |
| 38 | 123 |
| 40 | 92 |
| 41 | 111 |
| 42 | 111 |
| 45 | 94 |
| 47 | 128 |

Les composés suivant l'invention peuvent par conséquent être utilisés dans des compositions pharmaceutiques contenant en tant qu'ingrédient actif au moins un composé de la formule générale I, ou un sel d'addition d'un tel composé avec l'acide chlorhydrique ou nitrique, ou avec l'hydroxyde de sodium ou de potassium, mélangé à un véhicule ou support physiologiquement acceptable.

Les composés de l'invention peuvent être administrés sous forme de dragées, de comprimés, de capsules, de suppositoires ou de solutions à injecter, la posologie journalière se situant entre 5 et 50 mg de composé actif.

La préparation des composés de la formule générale I est illustrée par les exemples ci-après.

Exemple 1

*Préparation de la 3-isopropylcarbamoyl-sulfonamido-4-(3-méthylphényl)amino-pyridine* (Formule I: $R_1$=H; $R_2$=3-méthylphényle; $R_3$=H; $R_4$=isopropyle).

A. Préparation de la 3-sulfonamido-4-(3-méthylphényl)amino-pyridine.

Le mélange de 0,01 mole de 3-sulfonamido-4-chloro-pyridine, de 0,02 mole de 3-méthylphénylamine et de 50 ml d'éthanol anhydre est chauffé pendant 9 heures à la température de reflux. Après élimination de l'éthanol par distillation, le résidu est repris dans un excès d'hydroxyde de sodium dilué et l'amine en excès est extraite à l'aide d'éther.

La solution aqueuse est ensuite décolorée par un traitement au charbon actif et filtrée, le filtrat étant neutralisé par l'acide acétique, le précipité formé séparé par filtration et purifié par recristallisation dans un mélange d'eau et d'acétone. Les cristaux obtenus de couleur brun clair ont un point de fusion de 184—186°C.

B. Préparation de la 3-isopropylcarbamoyl-sulfonamido-4-(3-méthylphényl)amino-pyridine.

On fait réagir 0,01 mole de 3-sulfonamido-4-(3-méthylphényl)-amino-pyridine et 0,015 mole d'isocyanate d'isopropyle en présence de 0,02 mole de triéthylamine et de 20 ml de dichlorométhane pendant 20 heures à la température ordinaire.

Après évaporation sous vide, le résidu est repris dans un excès de carbonate de sodium en solution diluée et après filtration, le filtrat est acidifié par l'acide acétique.

Le précipité formé est séparé par filtration et lavé à plusieurs reprises avec de l'eau refroidie à la température de la glace.

On obtient le composé indiqué en tête sous forme d'une poudre blanche d'un point de fusion de 147—149°C.

Cette réaction peut également être réalisée en l'absence d'un solvant par chauffage des réactifs sur un bain marie.

Exemple 2

*Préparation du nitrate de 3-isopropyl-carbamoyl-sulfonamido-4-pentylamino-pyridine* (Formule I: $R_1$=H; $R_2$=$-(CH_2)_4-CH_3$; $R_3$=H; $R_4$=isopropyle)

En agitant, on fait réagir 0,01 mole du sel de sodium de la 3-sulfonamido-4-pentylaminopyridine avec 0,015 mole d'isocyanate d'isopropyle dans un mélange 3:2 d'eau et d'acétone jusqu'à disparition de l'odeur âcre de l'isocyanate. La solution ou suspension hydro-acétonique est ensuite traitée avec un excès d'acide nitrique concentré. Le précipité formé est séparé par filtration, lavé avec de l'eau refroidie à la température de la glace et recristallisé dans de l'eau contenant quelques gouttes d'acide nitrique. Le produit cristallisé possède un point de fusion de 156—158°C.

Exemple 3

*Préparation de la 3-(N-butyl-N-éthyl)-carbamoylsulfonamido-4-(3-méthylphényl) amino-pyridine* (Formule I: $R_1$=H; $R_2$=3-méthylphényle; $R_3$=éthyle; $R_4$=n.butyle)

On fait réagir pendant quelques heures 5 millimoles de l'éthylcarbamate de 3-aminosulfonyl-4-(3-méthylphényl) amino-pyridine avec 25 millimoles de N-éthyl, N-n butylamine, dans 30 ml de toluène anhydre en présence d'un tamis moléculaire d 4 Å. Après élimination du toluène par distillation, le résidu est repris dans un excès d'hydroxyde de sodium en solution diluée et l'amine en excès est extraite par de l'éther.

La solution aqueuse est acidifiée par de l'acide chlorhydrique dilué, le produit précipité est purifié par un traitement à l'hydrogénocarbonate de sodium et reprécipité par addition d'acide.

Le composé indiqué en tête possède un point de fusion de 133—135°C.

Exemple 4

*Préparation de la 3-(N-isopropyl-N-benzyl)-carbamoylsulfonamido-4-(3-méthylphényl)amino-pyridine* (Formule I: $R_1$=H; $R_2$=3-méthylphényle; $R_3$=isopropyle; $R_4$=benzyle)

Le mélange de 5 millimoles d'éthyl-carbamate de 3-aminosulfonyl-4-(3-méthylphényl)amino-pyridine et de 25 millimoles d'isopropylbenzyl amine dans du toluène ou du chloroforme anhydre est chauffé pendant 20 heures dans l'autoclave à 100°C en présence d'un tamis moléculaire de 4 Å.

Isolé, purifié et cristallisé comme décrit à l'exemple 3, le composé indiqué en tête possède un point de fusion de 140—142°C.

Exemple 5

*Préparation de la 3-(N-tétraméthylène)-carbamoylsulfonamido-4-(3-méthylphényl) amino-pyridine.* (Formule I: $R_1$=H; R=3-méthylphényle; $R_3$ et $R_4$ forment ensemble $-CH_2-CH_2-CH_2-CH_2-$)

A. On chauffe pendant 45 heures à la température de reflux le mélange de 5 millimoles de 1,1-dioxyde de 3-oxo-4-(3-méthylphényl)-3,4-dihydro-1,2,4-pyrido[4,3-2]-thiadiazine et de 25 millimoles de pyrrolidine dans le toluène.

Après élimination du toluène par distillation, on reprend le résidu dans de l'eau et on acidifie par de l'acide chlorhydrique dilué. Le précipité formé est purifié dans de l'hydrogénocarbonate de sodium et reprécipité par addition d'acide. Le composé obtenu possède un point de fusion de 158—160°C.

B. En appliquant le deuxième procédé suivant l'invention, on obtient le composé indiqué en tête en faisant réagir l'éthyl-carbamate de la 3-aminosulfonyl-4-(3-méthylphényl)amino-pyridine suivant le mode opératoire de l'exemple 3 avec la pyrrolidine en présence d'un tamis moléculaire.

Exemples 6 à 8

Les composés repris dans le tableau II sont préparés par le mode opératoire de l'exemple 1.

Exemples 9 à 19

Les composés repris dans le tableau III sont préparés par le mode opératoire de l'exemple 2.

Exemples 20 à 44

Les composés repris dans le tableau IV sont préparés par le mode opératoire de l'exemple 3.

Exemples 45 à 50

Les composés repris dans le tableau V sont préparés par le mode opératoire de l'exemple 4.

TABLEAU II

Composé de formule I :

$R_1$ = hydrogène ; X = 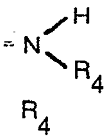

| Exemple No. | $R_2$ | $R_4$ | Point de fusion (°C) |
|---|---|---|---|
| 6 | 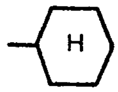 | 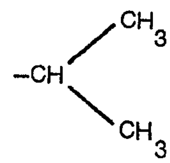 | 185 – 187 |
| 7 | $-(CH_2)_6-CH_3$ | 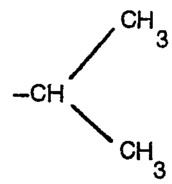 | 139 – 141 |
| 8 | 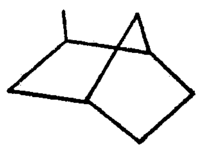 norbornyle | 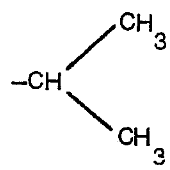 | 168 – 170 |

TABLEAU III

Composé de formule I :

$R_1$ = hydrogène ; X = 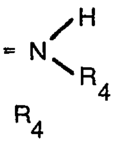

| Exemple No. | $R_2$ | $R_4$ | Point de fusion (°C) |
|---|---|---|---|
| 9 | 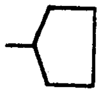 | 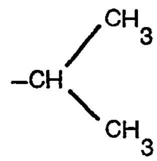 | 182 – 184 nitrate |
| 10 | $-(CH_2)_5-CH_3$ | 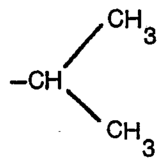 | 163 – 165 nitrate |
| 11 | 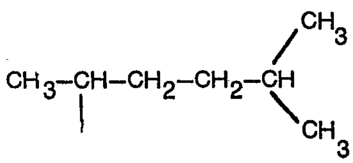 | 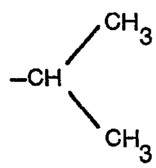 | 156 – 157 nitrate |
| 12 | 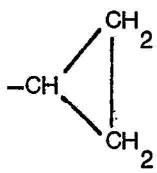 | 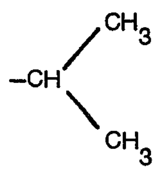 | 174 – 176 nitrate |
| 13 | 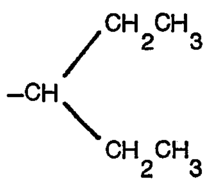 | 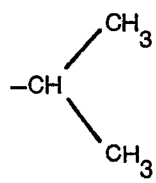 | 113 – 115 nitrate |
| 14 | 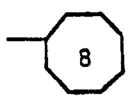 | 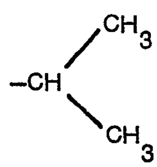 | 161 – 162 nitrate |
| 15 | 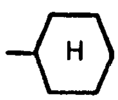 | $-CH_2-CH_3$ | 180 – 182 nitrate |
| 16 | 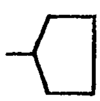 | $-CH_2-CH_3$ | 186 – 188 nitrate |
| 17 | 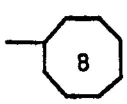 | $-CH_2-CH_3$ | 155 – 157 nitrate |

TABLEAU III (Suite)

Composé de formule I :

$R_1$ = hydrogène ; X = 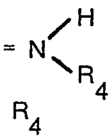

| Exemple No. | $R_2$ | $R_4$ | Point de fusion (°C) |
|---|---|---|---|
| 18 | 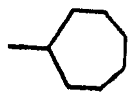 | 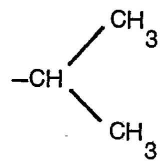 | 163 – 165 nitrate |
| 19 | $-CH_2CH_2CH_2Cl$ | 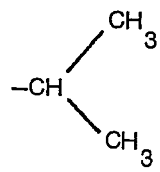 | 161 – 163 |

TABLEAU IV

| Exemple No. | | Composé de formule I: X = 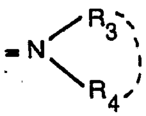 | | | Point de fusion (°C) |
|---|---|---|---|---|---|
| 20 | $R_1 = H$ | $R_2 = n, C_4H_9$ | 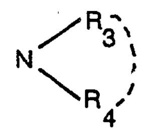 | = 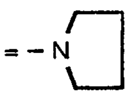 | 170 – 172 |
| 21 | $R_1 = H$ | $R_2 =$ 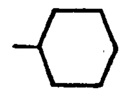 | 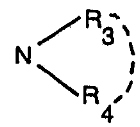 | = 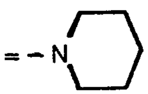 | 166 – 169 |
| 22 | $R_1 = C_2H_5$ | $R_2 = n, C_4H_9$ | 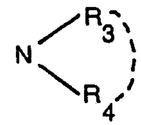 | = 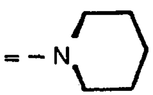 | 187 – 189 |
| 23 | $R_1 = C_6H_{11}$ | $R_2 = CH_2-CH=CH_2$ | 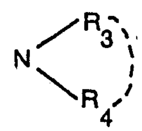 | = 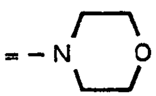 | 195 – 197 |
| 24 | $R_1 = \mu.C_4H_9$ | $R_2 = n, C_4H_9$ | 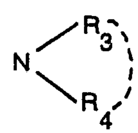 | = 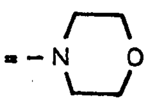 | 208 – 210 |
| 25 | $R_1 = H$ | $R_2 = CH_3-CH-CH_2-$ 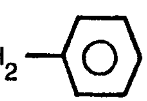 | 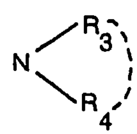 | = 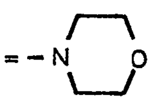 | 196 – 198 |

TABLEAU IV (Suite)

| Exemple No. | | | Composé de formule I : 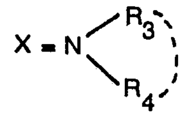 | | Point de fusion (°C) |
|---|---|---|---|---|---|
| 26 | $R_1 = C_2H_5$ | $R_2 = -CH_2$ 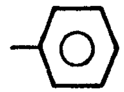 | 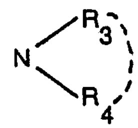 | = 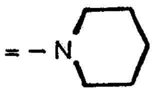 | 148 – 150 |
| 27 | $R_1 = H$ | $R_2$ = 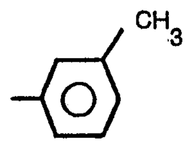 | $R_3 = C_2H_5$ | $R_4 = CH_2-CH_2OH$ | 137 – 139 |
| 28 | $R_1 = H$ | $R_2$ = 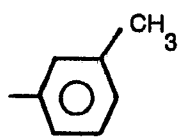 | $R_3 = -CH_2-CH=CH_2$ | $R_4 = -CH_2-CH=CH_2$ | 138 – 140 |
| 29 | $R_1 = H$ | $R_2$ = 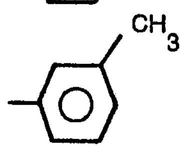 | 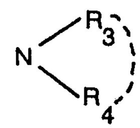 | = 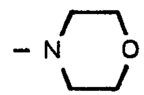 | 149 – 151 |
| 30 | $R_1 = H$ | $R_2$ = 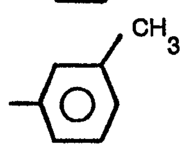 | 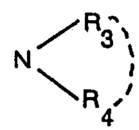 | = 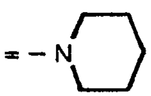 | 155 – 158 |
| 31 | $R_1 = H$ | $R_2$ = 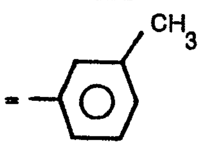 | 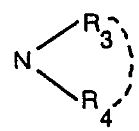 | = 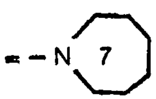 | 123 – 125 |

TABLEAU IV (Suite)

| Exemple No. | | Composé de formule I: X = 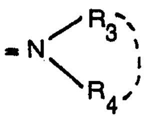 | | | Point de fusion (°C) |
|---|---|---|---|---|---|
| 32 | $R_1$ = H | $R_2$ = 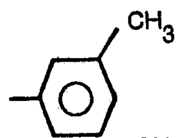 | 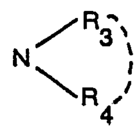 | = 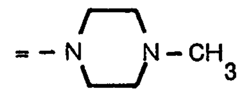 | 161 – 163 |
| 33 | $R_1$ = H | $R_2$ = 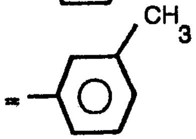 | $R_3$ = $C_2H_5$ | $R_4$ = 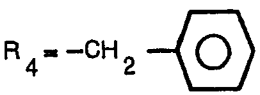 | 156 – 158 |
| 34 | $R_1$ = H | $R_2$ = 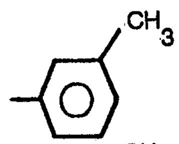 | 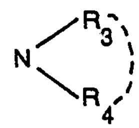 | = 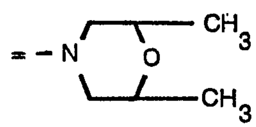 | 162 – 164 |
| 35 | $R_1$ = H | $R_2$ = 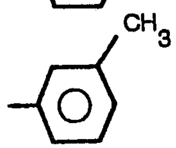 | 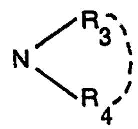 | = 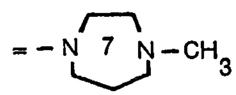 | 181 dichlorhydrate |
| 36 | $R_1$ = H | $R_2$ = 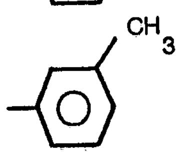 | 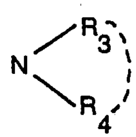 | = 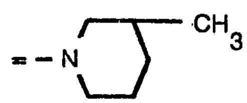 | 129 – 131 |
| 37 | $R_1$ = H | $R_2$ = 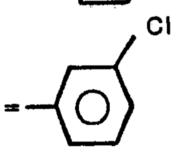 | 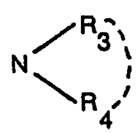 | = 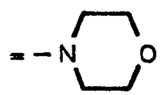 | 165 – 167 |

TABLEAU IV (Suite)

| Exemple No. | | Composé de formule I: X = 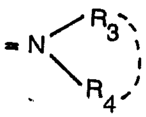 | | | Point de fusion (°C) |
|---|---|---|---|---|---|
| 38 | $R_1$ = H | $R_2$ = 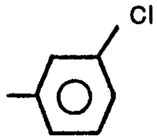 | 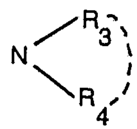 | = 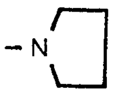 | 150 – 152 |
| 39 | $R_1$ = H | $R_2$ = 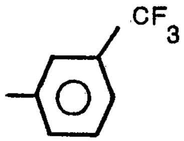 | 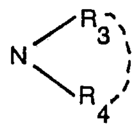 | = 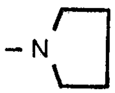 | 160 – 162 |
| 40 | $R_1$ = H | $R_2$ = 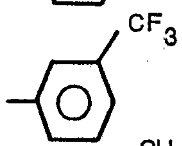 | 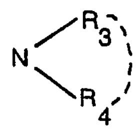 | = 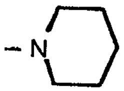 | 142 – 144 |
| 41 | $R_1$ = H | $R_2$ = 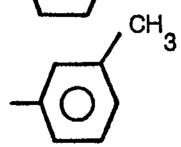 | 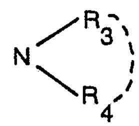 | = 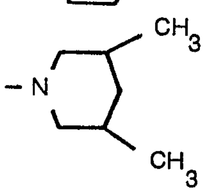 | 158 – 159 |
| 42 | $R_1$ = H | $R_2$ = 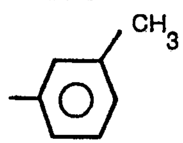 | 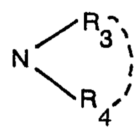 | = 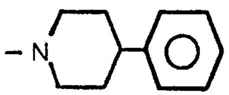 | 130 – 132 |
| 43 | $R_1$ = $CH_3$ | $R_2$ = $-CH_2-C\equiv CH$ | 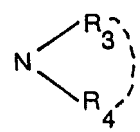 | = 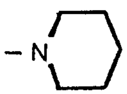 | 184 – 186 |
| 44 | $R_1$ = H | $R_2$ = 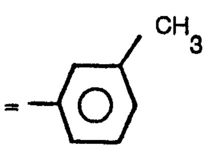 | 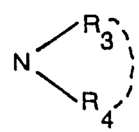 | = 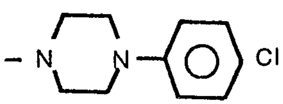 | 148 – 151 |

TABLEAU V

Composé de formule I:

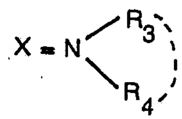

| Exemple No. | $R_1$ | $R_2$ | $R_3$ | $R_4$ | Point de fusion (°C) |
|---|---|---|---|---|---|
| 45 | H | 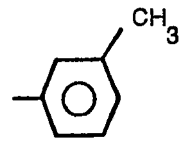 | $C_2H_5$ | $C_2H_5$ | 144 – 146 |
| 46 | H | 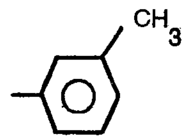 | $nC_4H_9$ | $nC_4H_9$ | 131 – 133 |
| 47 | H | 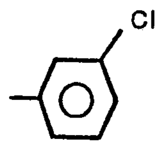 | $C_2H_5$ | $C_2H_5$ | 141 – 143 |
| 48 | H | 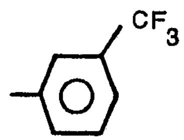 | $nC_4H_9$ | $nC_4H_9$ | 124 – 126 |
| 49 | $C_2H_5$ | $-CH_2$ 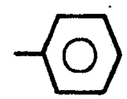 | 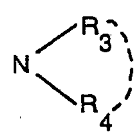 | = – 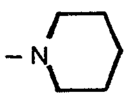 | 148 – 150 |
| 50 | H | 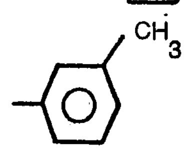 | 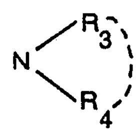 | = 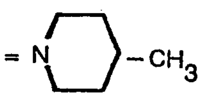 | 149 – 151 |

**Revendications**

1. Composés de la formule générale:

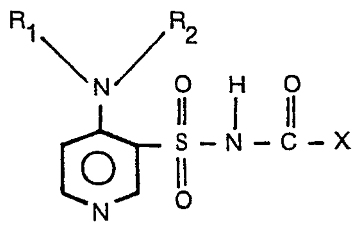

(I)

dans laquelle
$R^1$ désigne un atome d'hydrogène, un radical alkyle en $C_1$—$C_4$ à chaîne droite ou ramifiée ou le radical cyclohexyle, et
$R_2$ représente un radical alkyle en $C_1$—$C_7$ à chaîne droite ou ramifiée, un radical chloroalkyle en $C_1$—$C_3$, un radical cycloalkyle en $C_3$—$C_8$, le radical propényle ou propynyle, un radical phényle éventuellement substitué par un groupe méthyle, par un atome de chlore ou par un groupe trifluorométhyle, le radical benzyle, le radical 1-méthyl-2-phényl éthyle ou le radical norbornyle;
et X représente un radical de formule:

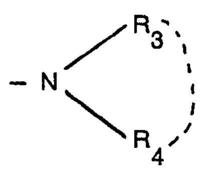

(IV)

où $R_3$ représente un atome d'hydrogène, un radical alkyle en $C_2$—$C_4$ à chaîne droite ou ramifiée ou propényle et
$R_4$ un radical alkyle en $C_1$—$C_4$ à chaîne droite ou ramifiée, un groupe propényle, hydroxyéthyle ou benzyle, ou $R_3$ et $R_4$ forment avec l'atome d'azote adjacent un groupe hétérocyclique azoté saturé en $C_4$—$C_6$ pouvant contenir un deuxième atome d'azote ou un autre hétéroatome et éventuellement substitué par un ou deux groupes méthyle ou un groupe phényle ou chlorophényle, étant entendu que
$R_1$ et $R_2$ peuvent prendre toutes les valeurs indiquées lorsque le radical

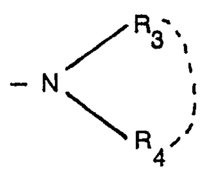

représente une fonction amine tertiaire, hétérocyclique ou di-N-substituée par des groupes en $C_2$ au moins, et que $R_1$, $R_3$, $R_4$ peuvent prendre toutes les valeurs indiquées lorsque $R_2$ représente un radical alkyle en $C_5$—$C_7$, à chaîne droite ou ramifiée, un radical cycloalkyle en $C_3$—$C_8$, un groupe chloroalkyle en $C_1$—$C_3$ ou le groupe norbornyle; ainsi que les sels d'addition d'acides physiologiquement acceptables de ces composés.

2. Composés selon la revendication 1, caractérisés en ce que $R_3$ représente un radical alkyle en $C_2$—$C_3$, à chaîne droite ou ramifiée ou un groupe propényle, et
$R_4$ représente un radical alkyle en $C_2$—$C_4$ à chaîne droite ou ramifiée ou un groupe propényle ou hydroxyéthyle, ou $R_3$ et $R_4$ forment avec l'atome d'azote adjacent un groupe pyrrolidino, pipéridino, morpholino, tétrahydroazépino, pipérazino, diméthylmorpholino, tétrahydrodiazepino, N-méthyltétrahydrodiazépino, méthylpipéridino, diméthylpipéridino, phénylpipéridino ou chlorophénylpipérazino.

3. Composés selon la revendication 1, caractérisé en ce que $R_1$ représente un atome d'hydrogène et $R_2$ un groupe alkyle en $C_5$—$C_7$, un groupe chloroalkyle en $C_1$—$C_3$ ou un groupe cycloalkyle en $C_3$—$C_8$.

## Claims

1. Compounds of the general formula:

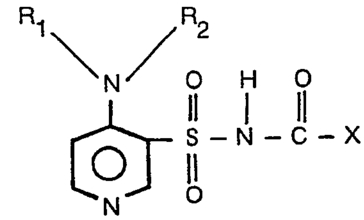

(I)

in which
$R_1$ represents a hydrogen atom, a $C_1$—$C_4$ straight or branched chain alkyl radical or the cyclohexyl radical, and
$R_2$ represents a $C_1$—$C_7$ straight or branched alkyl radical, a $C_1$—$C_3$ chloroalkyl radical, a $C_3$—$C_8$ cycloalkyl radical, the propenyl or propynyl radical, a phenyl radical optionally substituted by a methyl group, by a chlorine atom or by a trifluoromethyl group, the benzyl radical, the 1-methyl 2-phenyl ethyl radical or the norbornyl radical;
and X represents a radical of the formula:

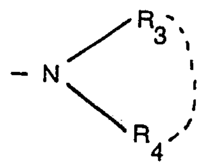

(IV)

in which
$R_3$ represents a hydrogen atom, a $C_2$—$C_4$ straight or branched chain alkyl or propenyl radical and $R_4$ a $C_1$—$C_4$ straight or branched alkyl radial, a propenyl, hydroxyethyl or benzyl group, or $R_3$ and $R_4$ form with the nitrogen atom to which they are bound, a $C_4$—$C_6$ saturated heterocyclic radical which may comprise a second nitrogen atom or another heteroatom and optionally substituted by one or two methyl groups or a phenyl or chlorophenyl group with the provisos that $R_1$ and $R_2$ may take all the aforesaid meanings when the radical

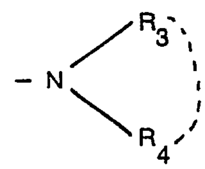

represents a tertiary, heterocyclic or amine function di-N-substituted by at least $C_2$ groups and that $R_1$, $R_2$, $R_1$ may take all the aforesaid meanings, when $R_2$ represents a $C_5$—$C_7$ straight or branched alkyl radical, a $C_3$—$C_8$ cycloalkyl radical, a $C_1$—$C_3$ chloroalkyl group: or the norbornyl group; as well as the physiologically acceptable addition salts of these compounds.

2. Compounds according to claim 1, characterized in that $R_3$ represents a $C_2$—$C_3$ straight or branched alkyl radical or a propenyl group, and
$R_4$ represents a $C_2$—$C_4$ straight or branched alkyl radical or a propenyl or hydroxyethyl group, or $R_3$ and $R_4$ form with the nitrogen atom to which they are bound, a pyrrolidino, piperidino, morpholino, tetrahydrodiazepino, piperazino, dimethylmorpholino, tetrahydrodiazepino, N-methyltetrahydrodiazepino, methylpiperidino, dimethylpiperidino, phenylpiperidino or chlorophenylpiperazino group.

3. Compounds according to claim 1, characterized in that $R_1$ represents a hydrogen atom and $R_2$ a $C_5$—$C_7$ alkyl group, a $C_1$—$C_3$ chloroalkyl group or a $C_3$—$C_8$ cycloalkyl group.

**Patentansprüche**

1. Verbindungen der allgemeinen Formel

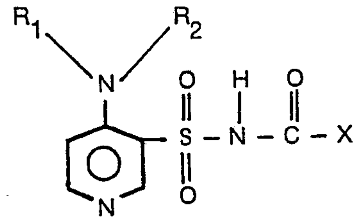

(I)

in der

$R_1$ ein Wasserstoffatom, einen geradkettigen oder verzweigten Alkylrest mit 1—4 C-Atomen oder einen Cyclohexylrest bedeutet und

$R_2$ einen geradkettigen oder verzweigten Alkylrest mit 1—7 C-Atomen, einen Chloralkylrest mit 1—3 C-Atomen, einen Cycloalkylrest mit 3—8 C-Atomen, einen Propenyl- oder Propinylrest, einen gegebenenfalls durch eine Methylgruppe, ein Chloratom oder eine Trifluormethylgruppe substituierten Phenylrest, einen Benzylrest, einen 1-Methyl-2-phenyläthylrest oder einen Norbornylrest bedeutet und

X einen Rest der Formel bedeutet

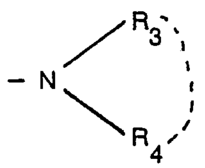

(IV)

worin $R_3$ ein Wasserstoffatom, einen geradkettigen oder verzweigten Alkylrest mit 2—4 C-Atomen oder einen Propenylrest bedeutet und

$R_4$ einen geradkettigen oder verzweigten Alkylrest mit 1—4 C-Atomen, einen Propenyl-, Hydroxyäthyl- oder Benzylrest bedeutet oder

$R_3$ und $R_4$ zusammen mit dem benachbarten Stickstoffatom einen stickstoffhaltigen, gesättigten heterocyclischen Rest mit 4—6 C-Atomen, der ein zweites Stickstoffatom oder ein anderes Heteroatom enthalten kann und gegebenenfalls durch eine oder zwei Methylgruppen oder eine Phenyl- oder Chlorphenylgruppe substituiert ist, bedeuten,

mit der Massgabe, dass

$R_1$ und $R_2$ alle angegebenen Bedeutungen haben können, wenn der Rest

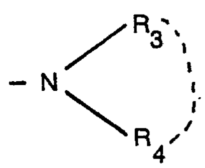

eine tertiäre Amingruppe, einen heterocyclischen Rest oder einen durch Gruppen mit mindestens zwei C-Atomen di-N-substituierten Rest bedeutet, und dass

$R_1$, $R_3$ und $R_4$ alle angegebenen Bedeutungen haben können, wenn $R_2$ einen geradkettigen oder verzweigten Alkylrest mit 5—7 C-Atomen, einen Cycloalkylrest mit 3—8 C-Atomen einen Chloralkylrest mit 1—3 C-Atomen oder einen Norbornylrest bedeutet,

sowie die Additionssalze dieser Verbindungen mit physiologisch verträglichen Säuren.

2. Verbindungen nach Anspruch 1, dadurch gekennzeichnet, dass $R_3$ einen geradkettigen oder verzweigten Alkylrest mit 2—3 C-Atomen oder eine Propenylgruppe bedeutet und $R_4$ einen geradkettigen oder verzweigten Alkylrest mit 2—4 C-Atomen oder eine Propenyl- oder Hydroxyäthylgruppe bedeutet oder

$R_3$ und $R_4$ mit dem benachbarten Stickstoffatom eine Pyrrolidino-, Piperidino-, Morpholino-, Tetrahydroazepino-, Piperazino-, Dimethylmorpholino-, Tetrahydrodiazepino-, N-Methyltetrahydrodiazepino-, Methylpiperidino-, Dimethylpiperidino-, Phenylpiperidino- oder Chlorphenylpiperazinogruppe bedeutet.

3. Verbindungen nach Anspruch 1, dadurch gekennzeichnet, dass $R_1$ ein Wasserstoffatom bedeutet und $R_2$ einen Alkylrest mit 5—7 C-Atomen, einen Chloralkylrest mit 1—3 C-Atomen oder einen Cycloalkylrest mit 3—8 C-Atomen bedeutet.